# EUROPEAN PATENT APPLICATION

(11) **EP 3 199 189 A1**
(43) Date of publication of application: **02.08.2017**
(21) Application number: 15845847.1
(22) Date of filing: 28.08.2015
(51) Int. Cl.: A61M 5/32, A61M 5/31, C08L 21/00

(54) **NOZZLE CAP**

(30) Priority: 02.10.2014 JP 2014204008
(71) Applicant: Sumitomo Rubber Industries, Ltd., Kobe-shi, Hyogo 651-0072 (JP)
(72) Inventor: MAEDA, Katsushi, Kobe-shi Hyogo 651-0072 (JP); TAHARA, Narihiro, Kobe-shi Hyogo 651-0072 (JP)
(74) Representative: Manitz Finsterwald Patentanwälte PartmbB
(86) International application number: PCT/JP2015/074528
(87) International publication number: WO 2016/052037

(57) **Abstract**

A nozzle cap (1) is provided, which is formed from a rubber composition containing a rubber component including a diene rubber and a non-diene rubber, wherein the diene rubber is present in a proportion of 20 to 70 parts by mass based on 100 parts by mass of the total of the rubbers in the rubber composition. The nozzle cap (1) has a moderate gas transmission property and, hence, can be quickly sterilized by EOG sterilization and steam sterilization, quickly removed of residues by deaeration, and quickly dried in a shorter period after the steam sterilization. In addition, the nozzle cap (1) is less liable to be loosened due to increase in internal pressure and compression set and detached from the nozzle, and has a reduced leaching substance amount before and after the sterilization.

## Description

### TECHNICAL FIELD

The present invention relates to a rubber nozzle cap for a prefilled syringe.

### BACKGROUND ART

In recent years, prefilled syringes which include a sterilized syringe barrel prefilled with a liquid drug tend to be increasingly used for prevention of medical malpractice, for handling ease and for improvement of cleanliness and the like.

A nozzle cap is attached to a distal end of a nozzle of the syringe barrel of such a prefilled syringe for liquid tightness, gas tightness, asepticity and the like.

The prefilled syringes include needle-equipped syringes configured such that a needle is preliminarily implanted in the nozzle of the syringe barrel, and needle-unequipped syringes configured so that, after the nozzle cap is removed, a needle is attached to the syringe for use. The nozzle cap includes a needle shield type having a needle sticking portion for the needle-equipped syringe and adapted to be fitted on the nozzle with the needle being stuck to a depth of several millimeters into the needle sticking portion, and a type adapted to be fitted on the nozzle of the needle-unequipped syringe (see PTL 1 to PTL 4).

The nozzle cap is designated by various names such as needle cap, needle shield, rubber cap, tip cap, plunger tip and syringe seal plug, according to the fitting arrangement and the like.

The prefilled syringe is produced by attaching the nozzle cap to the nozzle of the syringe barrel yet to be filled with the liquid drug, sterilizing the syringe barrel with ethylene oxide gas (EOG), steam, radioactive radiation such as a gamma ray, aseptically filling the syringe barrel with the liquid drug, and plugging the syringe barrel with a sterilized gasket, and then packaged to be shipped as a product.

The needle-equipped syringe, for example, is required to be configured so that, in the EOG or steam sterilization, the EOG or the steam permeates the rubber nozzle cap into the inside of the nozzle cap, and the entire needle including a tip portion stuck into the needle sticking portion, the nozzle and the like can be sterilized with the EOG or the steam.

Further, the nozzle cap is required to be configured so that the ethylene oxide gas used for the EOG sterilization and residues such as ethylene glycol and ethylene chlorohydrin generated as secondary products can be quickly removed after the EOG sterilization, and adsorbed moisture can be quickly dried off after the steam sterilization.

The nozzle cap is required to satisfy requirements specified in "3. Leaching Substance Test" of "7.03 Test Method for Infusion Rubber Plug" in the Japanese Pharmacopoeia 16th Edition before and after the sterilization.

Particularly, the nozzle cap is required to satisfy a requirement that the consumption of potassium permanganate reducing substances is not greater than 2.0 ml as specified in "3.5 Potassium Permanganate Reducing Substances."

Generally usable examples of a material for the nozzle gap include diene rubbers such as isoprene rubber (IR), butadiene rubber (BR) and styrene butadiene rubber (SBR), and non-diene rubbers such as butyl rubber and ethylene propylene rubber.

Particularly, the diene rubbers such as IR, BR and SBR are preferred, because the diene rubbers are excellent in needle stick sealability for prevention of liquid leakage from the tip of the needle particularly when the needle is stuck into the needle sticking portion. Further, the diene rubbers each have a higher gas transmission property and, therefore, are suitable as a material for the nozzle cap to be subjected to the EOG sterilization and the steam sterilization.

However, the diene rubbers tend to have a greater consumption of the potassium permanganate reducing substances than the non-diene rubbers, though satisfying the specified numerical requirement before the sterilization. The diene rubbers are substantially free from change in the consumption of the potassium permanganate reducing substances in the steam sterilization, but are liable to fail to satisfy the requirements in the leaching substance test in the presence of the residues such as ethylene oxide, ethylene glycol and ethylene chlorohydrin in the EOG sterilization.

Further, the diene rubbers are insufficient in ozone resistance. When a nozzle cap made of any of the diene rubbers is fitted on a nozzle having an outer size slightly greater than the inner diameter of the nozzle cap and stored with its diameter expanded for a long period of time, for example, the nozzle cap is problematically liable to suffer from cracking over time.

The problem associated with the cracking can be solved, for example, by combinational use of a wax and an amine anti-ozone stabilizer which bleed onto a surface of the nozzle cap to form a film on the surface. However, the use of the wax and the anti-ozone stabilizer is not preferred in consideration of the leaching substance amount and the discoloration of the nozzle cap.

On the other hand, the non-diene rubbers have a smaller leaching substance amount than the diene rubbers. Particularly, the butyl rubber is excellent in chemical resistance, heat resistance, ozone resistance and the like.

Problematically, however, the butyl rubber is not suitable as the material for the nozzle cap to be subjected to the EOG sterilization and the steam sterilization because of its very low gas transmission property.

That is, a nozzle cap made of the butyl rubber requires a longer period of time for the sterilization, for the removal of the residues by the deaeration after the EOG sterilization and for the drying after the steam sterilization. This is problematic in terms of the productivity of the prefilled syringe.

Further, the butyl rubber nozzle cap cannot quickly release an internal pressure occurring therein, for example, during the steam sterilization and the subsequent drying, or during the deaeration after the EOG sterilization. Problematically, therefore, the nozzle cap is liable to be loosened due to increase in internal pressure to be thereby detached from the nozzle.

Of the non-diene rubbers, the ethylene propylene rubber is excellent in chemical resistance, heat resistance and ozone resistance, but suffers from greater compression set. Therefore, when a nozzle cap made of the ethylene propylene rubber is fitted on a nozzle having an outer size slightly greater than the inner diameter of the nozzle cap and stored with its diameter expanded for a long period of time as described above, for example, the nozzle cap is problematically liable to have lower sealability with respect to the nozzle to suffer from liquid leakage or to be detached from the nozzle.

### CITATION LIST

### PATENT LITERATURE

PTL 1: JP-HEI10(1998)-248929A
PTL 2: JP-2013-43957A
PTL 3: JP-2004-532060A
PTL 4: WO2011/14917A1

### SUMMARY OF INVENTION

### TECHNICAL PROBLEM

It is an object of the present invention to provide a nozzle cap which has a moderate gas transmission property so that the nozzle cap can be quickly sterilized by the EOG sterilization or the steam sterilization, quickly removed of the residues by the deaeration, and quickly dried in a shorter period of time after the steam sterilization, is less liable to be loosened due to the increase in internal pressure and the compression set and detached from the nozzle, and satisfies the requirements in the predetermined leaching substance test with a reduced leaching substance amount before and after the sterilization.

### SOLUTION TO PROBLEM

According to the present invention, there is provided a nozzle cap for a prefilled syringe, the nozzle cap being formed from a rubber composition which contains a rubber component including a diene rubber and a non-diene rubber, wherein the diene rubber is present in a proportion of not less than 20 parts by mass and not greater than 70 parts by mass based on 100 parts by mass of the total of the rubbers in the rubber composition.

### ADVANTAGEOUS EFFECTS OF INVENTION

According to the present invention, the nozzle cap has a moderate gas transmission property so that the nozzle cap can be quickly sterilized by the EOG sterilization or the steam sterilization, quickly removed of the residues by the deaeration, and quickly dried in a shorter period of time after the steam sterilization. In addition, the nozzle cap is less liable to be loosened due to the increase in internal pressure and the compression set and detached from the nozzle, and satisfies the requirements specified in the leaching substance test by the Japanese Pharmacopoeia with a reduced leaching substance amount before and after the sterilization.

### BRIEF DESCRIPTION OF DRAWINGS

FIG. 1(a) is a sectional view illustrating an exemplary nozzle cap according to an embodiment of the present invention and a nozzle of a syringe barrel to be capped with the nozzle cap, and FIG. 1(b) is a sectional view showing a state in which the nozzle is capped with the nozzle cap.
FIG. 2(a) is a sectional view illustrating an exemplary nozzle cap according to another embodiment of the present invention and a nozzle of a syringe barrel to be capped with the nozzle cap, and FIG. 2(b) is a sectional view showing a state in which the nozzle is capped with the nozzle cap.

### DESCRIPTION OF EMBODIMENTS

The present invention provides a nozzle cap for a prefilled syringe. The nozzle cap is formed from a rubber composition which contains a rubber component including a diene rubber and a non-diene rubber. In the rubber composition, the diene rubber is present in a proportion of not less than 20 parts by mass and not greater than 70 parts by mass based on 100 parts by mass of the total of the rubbers.

According to the present invention, for use as the rubber component, the diene rubber which has a higher gas transmission property is blended in the aforementioned proportion with the non-diene rubber which is excellent in chemical resistance, heat resistance, ozone resistance and the like and has a smaller leaching substance amount. Thus, the nozzle cap is imparted with various excellent properties by utilizing the advantageous properties of the rubbers to achieve the object of the present invention.

More specifically, the nozzle cap has a moderate gas transmission property so that the nozzle cap can be quickly sterilized by the EOG sterilization or the steam sterilization, quickly removed of the residues by the deaeration, and quickly dried in a shorter period of time after the steam sterilization. In addition, the nozzle cap is less liable to be loosened due to the increase in internal pressure and the compression set and detached from the nozzle, and satisfies the requirements specified in the leaching substance test by the Japanese Pharmacopoeia with a reduced leaching substance amount before and after the sterilization.

### <Rubber Composition>

### (Diene Rubber)

Usable examples of the diene rubber include IR, BR, SBR, natural rubber (NR), chloroprene rubber (CR) and acrylonitrile butadiene rubber (NBR), which each have a double bond in a main chain thereof.

Particularly, the diene rubber is preferably at least one selected from the group consisting of IR, BR and SBR which are soft and excellent in needle stick sealability and each have a higher gas transmission property and a smaller leaching substance amount than the other diene rubbers.

Specific examples of the IR include Nipol (registered trade name) IR2200 and IR2200L available from Nippon Zeon Corporation, JSR (registered trade name) IR2200 available from JSR Co., Ltd., and CARIFLEX (registered trade name) IR0307K and IR0310K available from Kraton Performance Polymers Corporation, which may be used alone or in combination.

Specific examples of the BR include Nipol BR1220 and BR1250H available from Nippon Zeon Corporation, JSR BR01, JSR T700, JSR BR51 and JSR BR730 available from JSR Co., Ltd., and DIENE (registered trade name) NF35R available from Asahi Kasei Chemicals Corporation, which may be used alone or in combination.

For the SBR, S-SBR synthesized by a solution polymerization method is more preferred than E-SBR synthesized by an emulsion polymerization method for reduction of the leaching substance amount. A specific example of the S-SBR is S-SBR SE-0206 available from Sumitomo Chemical Co., Ltd.

### (Non-Diene Rubber)

Usable examples of the non-diene rubber include butyl rubber, ethylene propylene rubber, silicone rubber, fluororubber, urethane rubber, epichlorohydrin rubber and chlorinated polyethylene, which contain no double bond in a main chain thereof.

Particularly, the butyl rubber and the ethylene propylene rubber are preferred, which are excellent in chemical resistance, heat resistance, ozone resistance and the like and each have a smaller leaching substance amount.

Preferred examples of the butyl rubber include isobutylene-isoprene rubber (IIR) which is a copolymer of isobutylene and isoprene, chlorinated isobutylene-isoprene rubber (CIIR), brominated isobutylene-isoprene rubber (BIIR), brominated isobutylene-p-methylstyrene copolymer rubber and polyisobutylene which is a homopolymer of isobutylene. At least one of these butyl rubbers is preferably selected.

Specific examples of the IIR include Butyl 268 and Butyl 365 available from Japan Butyl Co., Ltd., and LANXESS (registered trade name) X_BUTYL 301 available from LANXESS Inc., which may be used alone or in combination.

Specific examples of the CIIR include CHLOROBUTYL 1066 available from Japan Butyl Co., Ltd., and LANXESS X_BUTYL CB1240 available from LANXESS Inc., at least one of which is used.

Specific examples of the BIIR include BROMOBUTYL 2255 available from Japan Butyl Co., Ltd, and LANXESS X_BUTYL BBX2 available from LANXESS Inc., at least one of which is used.

Specific examples of the brominated isobutylene-p-methylstyrene copolymer rubber include Exxpro (registered trade name) 3035 available from Exxon Mobile Corporation.

Specific examples of the polyisobutylene include OPPNOL B50SF, OPPNOL B80, OPPNOL B100, OPPNOL B150 and OPPNOL B200 available from BASF SE, which may be used alone or in combination.

The polyisobutylene is not crosslinked with the use of a crosslinking agent, and the leaching substance amount of the polyisobutylene is as small as that of the butyl rubber. Therefore, not greater than 20 mass % of the crosslinkable non-diene rubber may be replaced with the polyisobutylene.

On the other hand, preferred examples of the ethylene propylene rubber include ethylene propylene rubber (EPM) which is a copolymer of ethylene and propylene, and ethylene propylene diene rubber (EPDM) which is a copolymer of ethylene, propylene and a diene. At least one of these ethylene propylene rubbers is preferably used.

Of these ethylene propylene rubbers, a non-oil-extension and higher-diene-content type EPDM is preferred. Examples of the EPDM of this type include EPT4021, 4045, 4045M, 4070, 8030M, 9090M and X-4010M available from Mitsui Chemicals, Inc., which may be used alone or in combination.

### (Proportions of Rubbers to be blended)

In the present invention, the proportion of the diene rubber to be blended with the non-diene rubber need to be not less than 20 parts by mass and not greater than 70 parts by mass based on 100 parts by mass of the total of the rubbers.

The proportion of the non-diene rubber is a balance obtained by subtracting the proportion of the diene rubber from the total, i.e., not less than 30 parts by mass and not greater than 80 parts by mass.

If the proportion of the diene rubber is less than the aforementioned range, it will be impossible to provide the effect of the blending of the diene rubber for imparting the nozzle cap with the moderate gas transmission property, so that the resulting nozzle cap is not suitable for the EOG sterilization and the steam sterilization.

That is, a longer sterilization period will be required for the EOG sterilization and the steam sterilization. Further, a longer period of time will be required for the removal of the residues by the deaeration after the EOG sterilization and for the drying after the steam sterilization. This is problematic in terms of the productivity of the prefilled syringe.

Further, it will be impossible to quickly release an internal pressure occurring in the resulting nozzle cap, for example, during the steam sterilization and the subsequent drying, or during the deaeration after the EOG sterilization. Problematically, the nozzle cap is liable to be loosened due to increase in internal pressure to be thereby detached from the nozzle.

If the proportion of the diene rubber is greater than the aforementioned range, on the other hand, the resulting nozzle cap will have a reduced ozone resistance. Therefore, when the nozzle cap is fitted on a nozzle having an outer size slightly greater than the inner diameter of the nozzle cap and stored with its diameter expanded for a long period of time, for example, the nozzle cap is problematically liable to suffer from cracking over time.

It is known that ozone is present in a concentration of about 20 to about 50 ppb in the natural environment, and that ozone is generated, for example, from a static electricity removing apparatus in a medical product production environment. Therefore, the reduction in ozone resistance is problematic.

Further, the resulting nozzle cap may fail to satisfy the requirements specified in the leaching substance test due to the residues remaining after the EOG sterilization.

Where the diene rubber and the non-diene rubber are blended in the aforementioned proportions, in contrast, the nozzle cap is imparted with various excellent properties by utilizing the advantageous properties of the rubbers to achieve the object of the present invention.

For further improvement of the aforementioned effect, the proportion of the diene rubber is preferably not less than 25 parts by mass and not greater than 65 parts by mass based on 100 parts by mass of the total of the rubbers within the aforementioned range.

Where the nozzle cap is of the needle shield type for the needle-equipped syringe, it is preferred to add a liquid rubber (reactive plasticizer), a paraffin oil or a liquid polybutene oil to the rubber composition, or to adjust the type and the proportion of a reinforcement filler for improvement of the needle stick resistance with which the needle is stuck into the needle sticking portion, i.e., for reduction of the needle stick resistance.

Where the nozzle cap is for the needle-unequipped syringe, the formulation of the rubber composition can be determined without consideration of the needle stick resistance and the like.

The liquid rubber functioning as the reactive plasticizer may be added in a proportion of not greater than 20 parts by mass based on 100 parts by mass of the total of the diene rubber and the non-diene rubber to the rubber composition. Examples of the liquid rubber include liquid IRs such as KURAPRENE (registered trade name) LIR-30 and LIR-50 available from Kuraray Co., Ltd., and liquid BRs such as KURAPRENE LBR-300, LBR-305 and LBR-307 available from Kuraray Co., Ltd.

### (Crosslinking Component)

A crosslinking component for crosslinking the rubber component is blended in the rubber composition. Examples of the crosslinking component include a crosslinking agent, an accelerating agent and a crosslinking assisting agent (crosslinking activating agent).

Since the rubbers of the rubber component differ in crosslinking mechanism, agents optimum for the crosslinking are preferably selected and used in combination as the crosslinking component.

Where the diene rubber such as the BR and the CIIR (non-diene rubber) are used in combination as the rubber component, for example, two types of crosslinking agents, i.e., a peroxide crosslinking agent for crosslinking the diene rubber and a triazine crosslinking agent for crosslinking the CIIR, are preferably used in combination as the crosslinking component.

Any of various peroxide crosslinking agents which are generally used as a rubber crosslinking agent and are capable of generating peroxide radicals in a heating atmosphere or an oxidation-reduction atmosphere to crosslink the rubber is usable as the peroxide crosslinking agent. Particularly, a dialkyl peroxide is preferred.

Examples of the dialkyl peroxide include 2,5-dimethyl-2,5-di(tert-butylperoxy)hexane, 2,5-dimethyl-2,5-di(tert-butylperoxy)hexyne-3 and n-butyl-4,4-bis(tert-butylperoxy)valerate, which may be used alone or in combination.

Examples of the triazine crosslinking agent include 2-dibutylamino-4,6-dimercapto-s-triazine, 6- [bis (2-ethylhexyl) amino] -1, 3, 5-triazine-2, 4-dithiol, 6-diisobutylamino-1,3,5-triazine-2,4-dithiol and 6-diisopropylamino-1,3,5-triazine-2,4-dithiol, which may be used alone or in combination.

The proportion of the peroxide crosslinking agent to be blended is preferably not less than 0.1 part by mass and not greater than 2.0 parts by mass based on 100 parts by mass of the overall rubber component, and the proportion of the triazine crosslinking agent to be blended is preferably not less than 0.1 part by mass and not greater than 2.0 parts by mass based on 100 parts by mass of the overall rubber component.

If the proportion of even one of the crosslinking agents is less than the aforementioned range, it will be impossible to produce the nozzle cap having the required physical properties due to insufficient crosslinking. If the proportion of even one of the crosslinking agents is greater than the aforementioned range, on the other hand, the excess crosslinking agent is liable to influence the leaching substance amount.

A nitrosamine-free accelerating agent is preferred as the accelerating agent. Examples of the nitrosamine-free accelerating agent include a thiuram accelerating agent, a dithiocarbamate accelerating agent and a thiourea accelerating agent, at least one of which is preferably used. Particularly, these three types of accelerating agents are preferably used in combination.

Examples of the thiuram accelerating agent include tetrakis(2-ethylhexyl)thiuram disulfide (TOT-N) and tetrabenzylthiuram disulfide (TBZTD), at least one of which is used.

An example of the dithiocarbamate accelerating agent is zinc dibenzyldithiocarbamate (ZTC).

An example of the thiourea accelerating agent is N,N'-diethylthiourea (EUR).

The proportions of the accelerating agents to be blended are each preferably not less than 0.5 parts by mass and not greater than 3.0 parts by mass based on 100 parts by mass of the overall rubber component.

If the proportion of even one of the accelerating agents is less than the aforementioned range, it will be impossible to produce the nozzle cap having the required physical properties due to insufficient crosslinking. If the proportion of even one of the accelerating agents is greater than the aforementioned range, on the other hand, the excess accelerating agent is liable to influence the leaching substance amount.

Examples of the crosslinking assisting agent include zinc oxide (zinc white) and magnesium oxide. The proportion of the crosslinking assisting agent to be blended is preferably not less than 0.3 parts by mass and not greater than 10 parts by mass based on 100 parts by mass of the overall rubber component.

Where the diene rubber and the IIR (non-diene rubber) are used in combination as the rubber component, two types of crosslinking agents, i.e., a peroxide crosslinking agent for crosslinking the diene rubber and a resin crosslinking agent for crosslinking the IIR, and one or two or more of the aforementioned accelerating agents and the crosslinking assisting agents are preferably used in combination as the crosslinking component.

Examples of the resin crosslinking agent include alkylphenol disulfides and their lower polymers, alkylphenol formaldehyde resins, thermosetting phenol resins, phenol dialcohol resins, bisphenol resins and thermosetting bromomethyl alkylated phenol resins, which may be used alone or in combination.

Examples of the accelerating agent include a dithiocarbamic accelerating agent, a thiuram accelerating agent and a thiourea accelerating agent, which may be used alone or in combination.

Where the diene rubber and the BIIR (non-diene rubber) are used in combination as the rubber component, a sulfur crosslinking agent such as sulfur, the aforementioned resin crosslinking agent, and one or two or more of the dithiocarbamic accelerating agent, the thiuram accelerating agent and the thiourea accelerating agent are preferably used in combination as the crosslinking component.

Where the diene rubber and the EPM and/or the EPDM (non-diene rubber) are used in combination as the rubber component, only the peroxide crosslinking agent is used in a proportion of not less than 0.1 part by mass and not greater than 2.0 parts by mass based on 100 parts by mass of the overall rubber component as the crosslinking component.

If the proportion of the peroxide crosslinking agent is less than the aforementioned range, it will be impossible to produce the nozzle cap having the required physical properties due to insufficient crosslinking. If the proportion of the peroxide crosslinking agent is greater than the aforementioned range, on the other hand, the excess crosslinking agent is liable to influence the leaching substance amount.

### (Reinforcement Agent and Filler)

As in the conventional art, a reinforcement agent and a filler may be added to the rubber composition.

Preferred examples of the reinforcement agent include silica reinforcement agents such as hydrous silica and fumed silica which are white or pale-color reinforcement agents.

Specific examples of the hydrous silica include Nipsil (registered trade name) VN3, AQ, LP, ER and EL available from Tosoh Silica Corporation, which may be used alone or in combination.

Specific examples of the fumed silica include AEROSIL (registered trade name) 200 and AEROSIL R972 available from Japan Aerosil Co., Ltd., at least one of which is used.

The proportion of the silica to be added is preferably not less than 3 parts by mass and not greater than 50 parts by mass based on 100 parts by mass of the overall rubber component.

Examples of the filler include clay and talc.

Usable examples of the clay include baked clay and kaolin clay. Specific examples of the clay include SILLITIN (registered trade name) Z available from HOFFMANN MINERAL GmbH, SATINTONE (registered trade name) W and SATINTONE No. 5 available from ENGELHARD Corporation, NN KAOLIN CLAY and 5M KAOLIN CLAY available from Tsuchiya Kaolin Industry, Ltd., and PoleStar 200R available from Imerys Specialties Japan Co., Ltd., which may be used alone or in combination.

Specific examples of the talc include HIGH TORON A available from Takehara Kagaku Kogyo Co., Ltd., MICRO ACE (registered trade name) K-1 available from Nippon Talc Co., Ltd., MISTRON (registered trade name) VAPOR and VAPOR CB available from Imerys Specialties Japan Co., Ltd., which may be used alone or in combination.

The proportion of the filler to be added may be properly determined according to the types and combination of the rubbers of the rubber component and the intended rubber hardness of the nozzle cap.

However, it is not preferred to add a great amount of inorganic filler such as the clay and the talc to the rubber composition, because the inorganic filler is less dispersible and contains a very small amount of heavy metals.

In this case, a powdery filler such as of an olefin resin, a styrene elastomer or an ultrahigh molecular weight polyethylene (UHMWPE) may be added to the rubber composition.

### (Hydrotalcite and Coupling Agent)

A natural or synthetic hydrotalcite and/or a coupling agent may be added to the rubber composition.

The hydrotalcite functions as an acid acceptor which absorbs chlorine-containing gases and bromine-containing gases generated during the crosslinking of the CIIR and the BIIR to prevent crosslinking inhibition which may otherwise occur in the presence of these gases. Magnesium oxide described above also functions as the acid acceptor.

As well known, the coupling agent functions to improve the compatibility and the reactivity of the reinforcement agent and the filler such as silica, clay and talc with the rubber component to improve the reinforcement effect of the reinforcement agent and the filler.

Particularly, where the non-diene rubber is the CIRR or the BIIR, therefore, the hydrotalcite and the coupling agent are preferably used in combination. Where the non-diene rubber is the IIR alone, the hydrotalcite may be obviated.

Examples of the hydrotalcite include Mg-Al hydrotalcites such as Mg_{4.5}Al₂(OH)₁₃CO₃·3.5H₂O, Mg_{4.5}Al₂(OH)₁₃CO₃, Mg₄Al₂(OH)₁₂CO₃·3.5H₂O, Mg₆Al₂(OH)₁₆CO₃·4H₂O, Mg₅Al₂(OH)₁₄CO₃·4H₂O and Mg₃Al₂(OH)₁₀CO₃·1.7H₂O, which may be used alone or in combination.

A specific example of the hydrotalcite is DHT-4A (registered trade name) 2 available from Kyowa Chemical Industry Co., Ltd.

The proportion of the hydrotalcite to be added is preferably not greater than 5 parts by mass.

If the proportion of the hydrotalcite is excessively small, it will be impossible to sufficiently provide the aforementioned effect of the addition of the hydrotalcite.

Even if the proportion of the hydrotalcite is greater than the aforementioned range, it will be impossible to further enhance the intended effect. In addition, the leaching substance amount will be influenced.

Examples of the coupling agent include silane coupling agents such as vinyltriethoxysilane, vinyltriacetoxysilane, γ-mercaptopropyltrimethoxysilane, γ-methacryloxypropyltrimethoxysilane and γ-glycidoxyoxypropyltrimethoxysilane, and titanate coupling agents such as isopropyltriisostearoyl titanate, which may be used alone or in combination.

The proportion of the coupling agent to be added is preferably not less than 0.2 parts by mass and not greater than 3.0 parts by mass based on 100 parts by mass of the overall rubber component.

If the proportion of the coupling agent is less than the aforementioned range, it will be impossible to sufficiently provide the aforementioned effect of the addition of the coupling agent.

Even if the proportion of the coupling agent is greater than the aforementioned range, on the other hand, it will be impossible to further enhance the intended effect. In addition, the material costs will be increased.

### (Co-crosslinking Agent)

A co-crosslinking agent for assisting the crosslinking by the peroxide may be added in a proper proportion to the rubber composition.

Examples of the co-crosslinking agent include polyfunctional methacrylates and butadiene resins (syndiotactic 1-2-polybutadienes).

Examples of the polyfunctional methacrylates include trimethylolpropane trimethacrylate, trimethylolethane trimethacrylate, trimethylolpropane triacrylate, dipentaerythritolether hexaacrylate, pentaerythritol tetraacrylate and dimethylolpropane diacrylate, which may be used alone or in combination.

### (Other Ingredients)

A colorant such as titanium oxide and carbon black, and stearic acid, a polyethylene glycol, a low-density polyethylene (LDPE), an ethylene-vinyl acetate copolymer resin (EVA) and a chlorinated polyethylene functioning as a lubricant, a processing aid or a plasticizer may be added in proper proportions to the rubber composition.

### (Preparation of Rubber Composition)

The rubber composition containing the ingredients described above is prepared in a conventional manner. More specifically, the rubber component is prepared by blending the diene rubber and the non-diene rubber in the predetermined proportions and kneading the resulting mixture, and additives except the crosslinking component are added to and kneaded with the rubber component. Finally, the crosslinking component is added to and kneaded with the resulting mixture. Thus, the rubber composition is prepared.

An open roll, a sealed kneader, a Banbury mixer or the like is used for the kneading.

### <Nozzle Cap>

The inventive nozzle cap is produced by forming the rubber composition into a predetermined nozzle cap shape and then crosslinking the rubber composition.

### (Durometer Hardness)

The inventive nozzle cap preferably has a type-A durometer hardness of not less than 30 and not greater than 70 as measured in conformity with Japanese Industrial Standards JIS K6253-3_{:2012} "Rubber, vulcanized or thermoplastic - Determination of hardness - Part 3: Durometer method."

If the type-A durometer hardness is less than the aforementioned range, the nozzle cap is excessively soft, thereby influencing the working efficiency in attaching the nozzle cap to the nozzle and the handling ease in detaching the nozzle cap for use.

If the type-A durometer hardness is greater than the aforementioned range, on the other hand, the nozzle cap is excessively hard. Therefore, it will be difficult to fit the nozzle cap on the nozzle having a slightly greater outer size with the diameter of the nozzle cap expanded. Particularly, where the nozzle cap is of the needle shield type for the needle-equipped syringe, it will be difficult to stick the needle into the needle sticking portion of the nozzle cap so that the needle will be bent.

Where the type-A durometer hardness falls within the aforementioned range, in contrast, the nozzle cap has a moderate hardness and hence a proper sealability with respect to the nozzle. This prevents the nozzle cap from being loosened to be detached from the nozzle. With the hardness of the nozzle cap prevented from being excessively great, the nozzle cap can be easily fitted on the nozzle, and the needle can be easily stuck into the needle sticking portion of the nozzle cap.

Where the nozzle cap is of the needle shield type for the needle-equipped syringe, the type-A durometer hardness of the nozzle cap is preferably not greater than 55 within the aforementioned range in order to make it more easy to stick the needle into the needle sticking portion, and to more reliably prevent the nozzle from being bent.

Where the nozzle cap is for the needle-unequipped syringe, on the other hand, the type-A durometer hardness of the nozzle cap is preferably not less than 40 within the aforementioned range in order to further improve the sealability of the nozzle cap when the syringe is stored with the nozzle thereof capped with the nozzle cap, and to further more reliably prevent the nozzle cap from being loosened to be detached from the nozzle.

In order to control the type-A durometer hardness of the nozzle cap within the aforementioned range, the types, the grades and the proportions of the diene rubber, the non-diene rubber, the crosslinking component, the reinforcement agent, the filler, the lubricant, the processing aid, the plasticizer and other ingredients of the rubber composition may be properly adjusted.

### (Oxygen Transmission Rate)

A crosslinking product of the rubber composition for the inventive nozzle cap preferably has an oxygen transmission rate of not less than 5.8 × 10⁻⁹ cm³/ (cm²·s·Pa) (= 500 cm³/ (m²·day·bar)) and not greater than 2.32 × 10⁻⁸ cm³/ (cm²·s·Pa) (= 2000 cm³/ (m²·day·bar)) as measured per 1-mm thickness of a sample by Method B (isotactic pressure method) specified in Japanese Industrial Standards JIS K7126₋₁₉₈₇ "Gas transmission rate test method for plastic films and sheets."

Where the oxygen transmission rate falls within the aforementioned range, the nozzle cap has a moderate gas transmission property, permitting quick permeation of the EOG into the inside of the nozzle cap to sterilize the nozzle and the needle in a short period of time in the EOG sterilization. In the deaeration, the residues such as ethylene oxide, ethylene glycol and ethylene chlorohydrin can be quickly removed. Thus, the sterilization period is reduced to improve the productivity of the prefilled syringe.

Further, an internal pressure occurring in the nozzle cap during the deaeration can be quickly released, thereby preventing the nozzle cap from being loosened to be detached from the nozzle.

In order to control the oxygen transmission rate within the aforementioned range, the types, the grades and the proportions of the diene rubber and the non-diene rubber may be properly adjusted.

### (Water Vapor Transmission Rate)

The crosslinking product of the rubber composition for the inventive nozzle cap preferably has a water vapor transmission rate of not less than 0.1 g/(m²·24h) and not greater than 15 g/(m²·24h) as measured per 1-mm thickness of a sample at a temperature of 40°C at a relative humidity of 90 % by a measurement method specified in Japanese Industrial Standards JIS K7129:2008 "Plastics - Film and sheeting - Determination of water vapor transmission rate - Instrumental method."

Where the water vapor transmission rate falls within the aforementioned range, steam can quickly permeate the nozzle cap into the inside of the nozzle cap to sterilize the nozzle and the needle in a shorter period of time in the steam sterilization, and the nozzle and the needle can be quickly dried in a shorter period of time after the steam sterilization. This improves the productivity of the prefilled syringe.

Further, an internal pressure occurring in the nozzle cap during the steam sterilization and the subsequent drying can be quickly released, thereby preventing the nozzle cap from being loosened to be detached from the nozzle.

In order to control the water vapor transmission rate within the aforementioned range, the types, the grades and the proportions of the diene rubber and the non-diene rubber may be properly adjusted.

### (Ozone Resistance)

The crosslinking product of the rubber composition for the inventive nozzle cap preferably has an ozone resistance such that, even after the crosslinking product is maintained at an elongation percentage of 10 ± 1 % in a test environment having an ozone concentration of 50 ± 5 pphm and a temperature of 40°C as specified in International Standardization Organization Standards IS01431-1:2004 for 96 hours, the crosslinking product is free from cracking.

Thus, even if the nozzle cap is fitted on a nozzle having an outer size slightly greater than the inner diameter of the nozzle cap and stored with its diameter expanded for a long period of time, for example, the nozzle cap is less liable to suffer from cracking over time.

In order to impart the nozzle cap with the proper ozone resistance, the types, the grades and the proportions of the diene rubber and the non-diene rubber may be properly adjusted.

### (Nozzle Cap)

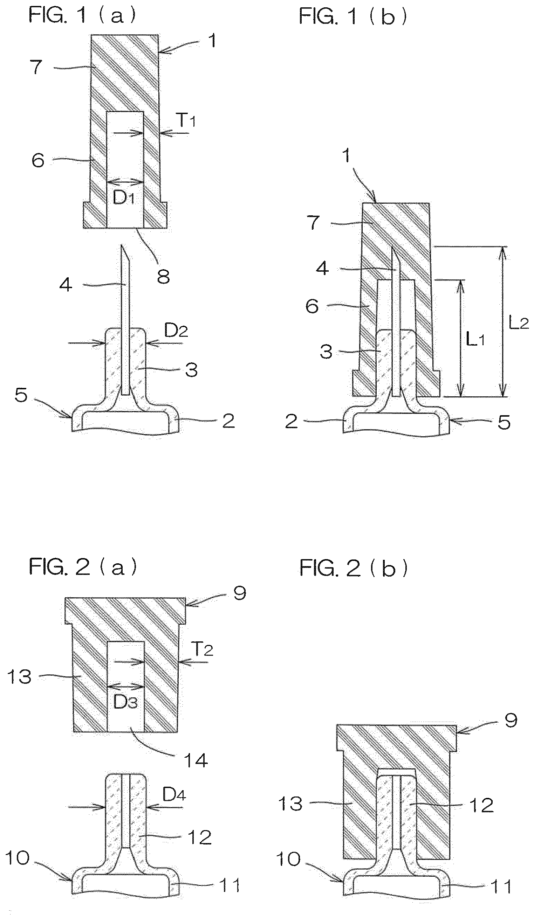

FIG. 1(a) is a sectional view illustrating an exemplary nozzle cap according to an embodiment of the present invention and a nozzle of a syringe barrel to be capped with the nozzle cap, and FIG. 1(b) is a sectional view showing a state in which the nozzle is capped with the nozzle cap.

Referring to these figures, the nozzle cap 1 according to this embodiment is for a needle-equipped syringe 5 including a syringe barrel 2 and a needle 4 preliminarily implanted in a nozzle 3 of the syringe barrel 2, and is entirely formed from the rubber composition described above. The nozzle cap 1 includes a tubular portion 6 having an inner diameter D₁ that is slightly smaller than the outer diameter D₂ of the nozzle 3, and a needle sticking portion 7 connected to one of opposite ends (an upper end in FIGS. 1(a) and 1(b)) of the tubular portion 6.

The needle sticking portion 7 has a columnar shape having an outer surface continuous to the tubular portion 6.

The tubular portion 6 has an opening 8 at the other end (a lower end in FIGS. 1(a) and 1(b)) thereof for receiving the nozzle 3 in the tubular portion 6 whereby the nozzle cap 1 is fitted on the nozzle 3. An axial dimension L₁ between the one end of the tubular portion 6 connected to and closed by the needle sticking portion 7 and the opening 8 provided at the other end of the tubular portion 6 satisfies a relationship L₁ < L₂ defined with respect to an axial dimension L2 between the opening 8 provided at the other end and a tip of the needle 4 with the nozzle cap 1 fitted on the nozzle 3. Thus, a tip portion of the needle 4 is stuck to a depth of about 5 mm into the needle sticking portion 7 for liquid-tight, gas-tight and aseptic sealing.

FIG. 2(a) is a sectional view illustrating an exemplary nozzle cap according to another embodiment of the present invention and a nozzle of a syringe barrel to be capped with the nozzle cap, and FIG. 2(b) is a sectional view showing a state in which the nozzle is capped with the nozzle cap.

Referring to these figures, the nozzle cap 9 according to this embodiment is adapted to be fitted on a nozzle 12 of a syringe barrel 11 of a needle-unequipped syringe 10, and is entirely formed from the rubber composition described above. The nozzle cap 9 includes a tubular portion 13 having an inner diameter D₃ that is slightly smaller than the outer diameter D₄ of the nozzle 12.

One of opposite ends (an upper end in FIGS. 2 (a) and 2(b)) of the tubular portion 13 is closed. The tubular portion 13 has an opening 14 at the other end (a lower end in FIGS. 2 (a) and 2(b)) thereof for receiving the nozzle 12 in the tubular portion 13 whereby the nozzle cap 9 is fitted on the nozzle 12.

The thickness T₁ of a minimum thickness portion of the tubular portion 6 of the nozzle cap 1 in FIGS. 1(a) and 1(b) and the thickness T₂ of a minimum thickness portion of the tubular portion 13 of the nozzle cap 9 in FIGS. 2(a) and 2(b) are each preferably 1.0 ± 0.5 mm.

If the thickness T₁, T₂ is greater than the aforementioned range, the gas permeation and the steam permeation will be suppressed, whereby a longer period of time will be required for the EOG sterilization and the deaeration or for the steam sterilization and the subsequent drying. This may reduce the productivity of the prefilled syringe.

Since an internal pressure occurring in the nozzle cap 1, 9 during the deaeration or during the steam sterilization and the subsequent drying cannot be quickly released, the nozzle cap 1, 9 is liable to be loosened due to increase in internal pressure to be thereby detached from the nozzle 3, 12.

If the thickness T₁, T₂ is smaller than the aforementioned range, on the other hand, the nozzle cap 1, 9 will be insufficient in rigidity. Therefore, the nozzle cap 1, 9 is liable to be improperly fitted on the nozzle 3, 12. This may reduce the productivity of the prefilled syringe.

Where the thickness T₁, T₂ falls within the aforementioned range, in contrast, the nozzle cap 1, 9 is imparted with a proper gas transmission property synergistically by controlling the oxygen transmission rate of the crosslinking product of the rubber composition for the nozzle cap 1, 9 within the aforementioned range. Particularly, in the EOG sterilization, the EOG can quickly permeate the nozzle cap 1, 9 into the inside of the nozzle cap 1, 9 to sterilize the nozzle 3, 12 and the needle 4 in a shorter period of time. In the deaeration, the residues such as ethylene oxide, ethylene glycol and ethylene chlorohydrin can be quickly removed. This improves the productivity of the prefilled syringe.

Further, it is possible to quickly release the internal pressure occurring in the nozzle cap 1, 9 during the deaeration, thereby preventing the nozzle cap 1, 9 from being loosened to be detached from the nozzle 3, 12.

Where the thickness T₁, T₂ falls within the aforementioned range and the water vapor transmission rate of the crosslinking product of the rubber composition for the nozzle cap 1, 9 is controlled within the aforementioned range, the steam can quickly permeate the nozzle cap 1, 9 into the inside of the nozzle cap 1, 9 to sterilize the nozzle 3, 12 and the needle 4 in a shorter period of time in the steam sterilization. Further, the drying subsequent to the steam sterilization can be quickly performed in a shorter period of time. This improves the productivity of the prefilled syringe.

Further, it is possible to quickly release the internal pressure occurring in the nozzle cap 1, 9 during the steam sterilization and the subsequent drying, thereby preventing the nozzle cap 1, 9 from being loosened to be detached from the nozzle 3, 12.

Further, it is possible to impart the nozzle cap 1, 9 with moderate rigidity, so that the nozzle cap 1, 9 can be properly fitted on the nozzle 3, 12. This improves the productivity of the prefilled syringe.

### (Production Method)

The nozzle caps according to the embodiments of the present invention described with reference to the figures can be produced by a conventional method by employing the aforementioned rubber composition as the material.

An uncrosslinked ribbon, pellet or sheet of the rubber composition is press-molded into a predetermined three-dimensional nozzle cap shape between an upper mold and a lower mold and crosslinked by pressure and heat, or the rubber composition is injection-molded into a predetermined three-dimensional nozzle cap shape and cross-linked in a mold.

Particularly, a press molding process using a vacuum press molding machine is preferred. In the press molding process, a plurality of nozzle caps can be produced on a single sheet of the rubber composition through so-called multi-cavity molding. This improves the productivity of the nozzle cap.

After the press molding, the sheet is coated with a silicone lubricant coating agent as required, and subjected to an exterior inspection step. Then, the nozzle caps are stamped out of the sheet, and subjected to a cleaning step, a sterilizing step, a drying step and a packaging step. Thus, the nozzle caps are produced.

Thereafter, the nozzle caps thus produced are each fitted on a nozzle of a syringe barrel yet to be filled with a liquid drug as described above and, in this state, subjected to the ethylene oxide gas (EOG) sterilization or the steam sterilization. Then, the syringe barrel is aseptically filled with the liquid drug and fitted with a sterilized gasket, and the resulting prefilled syringe is packaged to be shipped.

### EXAMPLES

### <Example 1>

### (Preparation of Rubber Composition)

For a rubber component, 30 parts by mass of BR (Nipol BR1220 available from Nippon Zeon Corporation) as the diene rubber and 70 parts by mass of CIIR (CHLOROBUTYL 1066 available from Japan Butyl Co., Ltd.) as the non-diene rubber were used.

Then, 20 parts by mass of a synthetic silica (Nipsil VN3 available from Tosoh Silica Corporation), 20 parts by mass of a clay (SATINTONE (registered trade name) W available from ENGELHARD Corporation), and ingredients shown below in Table 1 except the crosslinking component were blended with 100 parts by mass of the rubber component. The resulting mixture was kneaded at a filling percentage of 75 % in a 10 L pressurized sealed kneader, and aged at a room temperature. Further, the crosslinking component was added to and kneaded with the mixture by means of an open roll. Thus, a rubber composition was prepared.

**Table 1**

| Ingredients | Parts by mass |
|---|---|
| LDPE | 2 |
| Coupling agent | 1 |
| Paraffin oil | 5 |
| Peroxide crosslinking agent | 0.5 |
| Triazine crosslinking agent | 1 |
| Co-crosslinking agent TMTP | 0.5 |

The ingredients shown in Table 1 are as follows. The amounts (parts by mass) shown in Table 1 are based on 100 parts by mass of the overall rubber component. LDPE: HIGHWAX 220P available from Mitsui Chemicals, Inc.
Coupling agent: γ-mercaptopropyltrimethoxysilane
Peroxide crosslinking agent: 2,5-dimethyl-2,5-di(tert-butylperoxy)hexane
Triazine crosslinking agent: 2-dibutylamino-4,6-dimercapto-s-triazine
Co-crosslinking agent TMTP: trimethylolpropane trimethacrylate

Further, proper amounts of magnesium oxide, synthetic hydrotalcite, stearic acid, colorants (titanium oxide and carbon black) were added to the rubber composition.

### (Production of Nozzle Cap)

An unvulcanized sheet of the rubber composition was held between an upper mold and a lower mold at 170°C for 15 minutes to be molded by a vacuum press molding process and crosslinked. Thus, a plurality of nozzle caps 1 for the needle-equipped syringe 5 shown in FIGS. 1(a) and 1(b) were sequentially formed on the sheet.

Subsequently, a silicone lubricant coating agent was applied on opposite surfaces of the sheet, and then the sheet was subjected to an exterior inspection step, a stamping step, a cleaning step, a sterilizing step and a packaging step. Thus, the nozzle caps 1 were produced.

### <Example 2>

A rubber composition was prepared in substantially the same manner as in Example 1, except that 50 parts by mass of BR (Nipol BR1220 available from Nippon Zeon Corporation) as the diene rubber and 50 parts by mass of CIIR (CHLOROBUTYL 1066 available from Japan Butyl Co., Ltd.) as the non-diene rubber were used for the rubber component, and the crosslinking agents and the co-crosslinking agent were used in proportions shown in Table 2. Then, nozzle caps were produced in the same manner as in Example 1 by using the rubber composition.

### <Example 3>

A rubber composition was prepared in substantially the same manner as in Example 1, except that 50 parts by mass of IR (Nipol IR2200 available from Nippon Zeon Corporation) as the diene rubber and 50 parts by mass of CIIR (CHLOROBUTYL 1066 available from Japan Butyl Co., Ltd.) as the non-diene rubber were used for the rubber component, and the crosslinking agents and the co-crosslinking agent were used in proportions shown in Table 2. Then, nozzle caps were produced in the same manner as in Example 1 by using the rubber composition.

### <Example 4>

A rubber composition was prepared in substantially the same manner as in Example 1, except that 50 parts by mass of SBR (S-SBR SE-0206 available from Sumitomo Chemical Co., Ltd.) as the diene rubber and 50 parts by mass of CIIR (CHLOROBUTYL 1066 available from Japan Butyl Co., Ltd.) as the non-diene rubber were used for the rubber component, and the crosslinking agents and the co-crosslinking agent were used in proportions shown in Table 2. Then, nozzle caps were produced in the same manner as in Example 1 by using the rubber composition.

### <Example 5>

A rubber composition was prepared in substantially the same manner as in Example 1, except that 60 parts by mass of SBR (S-SBR SE-0206 available from Sumitomo Chemical Co., Ltd.) as the diene rubber, 35 parts by mass of EPDM (EPT4021 available from Mitsui Chemicals, Inc.) and 5 parts by mass of a polyisobutylene (OPPNOL B100 available from BASF SE) as the non-diene rubber were used for the rubber component, and the crosslinking agents and the co-crosslinking agent were used in proportions shown in Table 2. Then, nozzle caps were produced in the same manner as in Example 1 by using the rubber composition.

### <Comparative Example 1>

A rubber composition was prepared in substantially the same manner as in Example 1, except that 85 parts by mass of BR (Nipol BR1220 available from Nippon Zeon Corporation) as the diene rubber and 15 parts by mass of CIIR (CHLOROBUTYL 1066 available from Japan Butyl Co., Ltd.) as the non-diene rubber were used for the rubber component, and the crosslinking agents and the co-crosslinking agent were used in proportions shown in Table 3. Then, nozzle caps were produced in the same manner as in Example 1 by using the rubber composition.

### <Comparative Example 2>

A rubber composition was prepared in substantially the same manner as in Example 1, except that 15 parts by mass of BR (Nipol BR1220 available from Nippon Zeon Corporation) as the diene rubber and 85 parts by mass of CIIR (CHLOROBUTYL 1066 available from Japan Butyl Co., Ltd.) as the non-diene rubber were used for the rubber component, and the crosslinking agents and the co-crosslinking agent were used in proportions shown in Table 3. Then, nozzle caps were produced in the same manner as in Example 1 by using the rubber composition.

### <Comparative Example 3>

A rubber composition was prepared in substantially the same manner as in Example 1, except that 85 parts by mass of BR (Nipol BR1220 available from Nippon Zeon Corporation) as the diene rubber, and 10 parts by mass of EPDM (EPT4021 available from Mitsui Chemicals, Inc.) and 5 parts by mass of a polyisobutylene (OPPNOL B100 available from BASF SE) as the non-diene rubber were used for the rubber component, and the crosslinking agents and the co-crosslinking agent were used in proportions shown in Table 3. Then, nozzle caps were produced in the same manner as in Example 1 by using the rubber composition.

### <Comparative Example 4>

A rubber composition was prepared in substantially the same manner as in Example 1, except that 100 parts by mass of IR (Nipol IR2200 available from Nippon Zeon Corporation) was used alone as the diene rubber for the rubber component, and the crosslinking agents and the co-crosslinking agent were used in proportions shown in Table 3. Then, nozzle caps were produced in substantially the same manner as in Example 1 by using the rubber composition, except that the press molding was performed at 160°C for 15 minutes.

### <Comparative Example 5>

A rubber composition was prepared in substantially the same manner as in Example 1, except that 85 parts by mass of SBR (SBR SE-0206 available from Sumitomo Chemical Co., Ltd.) as the diene rubber, and 10 parts by mass of EPDM (EPT4021 available from Mitsui Chemicals, Inc.) and 5 parts by mass of a polyisobutylene (OPPNOL B100 available from BASF SE) as the non-diene rubber were used for the rubber component, and the crosslinking agents and the co-crosslinking agent were used in proportions shown in Table 3. Then, nozzle caps were produced in the same manner as in Example 1 by using the rubber composition.

### <Measurement of Durometer Hardness>

The type-A durometer hardness of each of the nozzle caps produced in Examples and Comparative Examples was measured by the aforementioned measurement method specified in JIS K6253-3_{:2002}.

### <Leaching Substance Test>

The leaching substance test specified in "7.03 Test Method for Infusion Rubber Plug" in the Japanese Pharmacopoeia 16th Edition was performed on each of the nozzle caps produced in Examples and Comparative Examples. A test liquid was obtained by immersing each of the nozzle caps in 10 g of water per 1 g of the nozzle cap in an autoclave at 121°C for 1 hour for extraction. Requirements are as follows:

### (Requirements)

State of test liquid: Colorless and transparent UV transmittance: Atransmittance of not less than 99.0 % at wavelengths of 430 nm and 650 nm
UV absorption spectrum: An absorbance of not greater than 0.20 in a wavelength range of 250 nm to 350 nm
Foaming: Deformed in 3 minutes
pH: A different in pH between the test liquid and a blank test liquid was in a range of ± 1.0
Zinc: Not greater than 1 µg/ml
Potassium permanganate reducing substances: Not greater than 2.0 ml/100 ml
Residues after evaporation: Not greater than 2.0 mg

A nozzle cap satisfying all the aforementioned requirements was rated as acceptable (o), and a nozzle cap failing to satisfy even one of the requirements is rated as unacceptable (×).

The test was performed on the nozzle caps produced by the production process up to the packaging step (before sterilization), nozzle caps subjected to the EOG sterilization under the following conditions (after EOG sterilization), and nozzle caps subjected to the steam sterilization under the following conditions (after steam sterilization).

### (Conditions for EOG Sterilization)

Gas concentration: 20 % EOG and 80 % carbon dioxide gas
Pressure: 98.1 kPa (= 1 kg/cm²)
Temperature: 50°C
Relative humidity: 50 %
Period: 5 hours

The nozzle caps of Comparative Example 2 were insufficient in oxygen transmission rate and, therefore, were not subjected to the EOG sterilization. Accordingly, the post-sterilization evaluations were not performed.

### (Conditions for Steam Sterilization)

Sterilization: At 123°C for 45 minutes
Drying: At 100°C for 5 hours
<Measurement of Oxygen Transmission Rate>

Sheets formed by press-molding and crosslinking the rubber compositions prepared in Examples and Comparative Examples were each used as a sample. The oxygen transmission rate of the sample was measured at a temperature of 40°C by the aforementioned measurement method specified in JIS K7126₋₁₉₃₇. A sample having an oxygen transmission rate per 1-mm thickness of not less than 5.8 × 10⁻⁹ cm³/ (cm²·s·Pa) and not greater than 2.32 × 10⁻⁸ cm³/ (cm²·s·Pa) was rated as acceptable (o), and a sample having an oxygen transmission rate falling outside this range was rated as unacceptable (×).

### <Measurement of Water vapor Transmission Rate>

Sheets formed by press-molding and crosslinking the rubber compositions prepared in Examples and Comparative Examples were each used as a sample. The water vapor transmission rate of the sample was measured at a temperature of 40°C at a relative humidity of 90 % by the aforementioned measurement method specified in JIS K7129_{:2008}. A sample having a water vapor transmission rate per 1-mm thickness of not less than 0.1 g/(m²·24h) and not greater than 15 g/(m²·24h) was rated as acceptable (o), and a sample having a water vapor transmission rate falling outside this range was rated as unacceptable (×).

### <Ozone Resistance Test>

Strips formed by press-molding and crosslinking the rubber compositions prepared in Examples and Comparative Examples and each having a thickness of 2 ± 0.2 mm and a width of about 10 mm were each used as a sample. The sample was maintained at an elongation percentage of 10 % in an environment having an ozone concentration of 50 ± 5 pphm, a temperature of 40°C and an air flow rate of about 22 mm/second as specified in ISO1431-1:2004 for 96 hours, and then checked for cracking. The number n of strips for each sample was 3. A sample with all the strips free from the cracking was rated as having an acceptable ozone resistance (○), and a sample with even one of the strips suffering from the cracking was rated as having an unacceptable ozone resistance (×).

The results of the above are shown in Tables 2 and 3.

**Table 2**

| | | Example 1 | Example 2 | Example 3 | Example 4 | Example 5 |
|---|---|---|---|---|---|---|
| Parts by mass | | | | | | |
| Diene rubber | BR | 30 | 50 | - | - | - |
| | IR | - | - | 50 | - | - |
| | SBR | - | - | - | 50 | 60 |
| Non-diene rubber | CIIR | 70 | 50 | 50 | 50 | - |
| | EPDM | - | - | - | - | 35 |
| | Polyisobutylene | - | - | - | - | 5 |
| Peroxide crosslinking agent | | 0.5 | 0.5 | 1 | 0.5 | 0.5 |
| Triazine crosslinking agent | | 1 | 0.8 | 0.8 | 0.8 | - |
| Co-crosslinking agent | | 0.5 | 0.5 | 1 | 0.5 | 0.5 |
| Evaluation | | | | | | |
| Type-A durometer hardness | | 49 | 53 | 46 | 51 | 55 |
| Leaching substance test | Before sterilization | ○ | ○ | ○ | ○ | ○ |
| | After EOG sterilization | ○ | ○ | ○ | ○ | ○ |
| | After steam sterilization | ○ | ○ | ○ | ○ | ○ |
| Oxygen transmission rate | | ○ | ○ | ○ | ○ | ○ |
| Water vapor transmission rate | | ○ | ○ | ○ | ○ | ○ |
| Ozone resistance | | ○ | ○ | ○ | ○ | ○ |

**Table 3**

| | | | Comparative Example 1 | Comparative Example 2 | Comparative Example 3 | Comparative Example 4 | Comparative Example 5 |
|---|---|---|---|---|---|---|---|
| Parts by mass | | | | | | | |
| Diene rubber | | BR | 85 | 15 | 85 | - | - |
| | | IR | - | - | - | 100 | - |
| | | SBR | - | - | - | - | 85 |
| Non-diene rubber | | CIIR | 15 | 85 | - | - | - |
| | | EPDM | - | - | 10 | - | 10 |
| | | Polyisobutylene | - | - | 5 | - | 5 |
| Peroxide crosslinking agent | | | 0.5 | 0.5 | 0.5 | 1 | 0.5 |
| Triazine crosslinking agent | | | 0.3 | 1 | - | - | - |
| Co-crosslinking agent | | | 0.5 | 0.5 | 0.5 | 1 | 0.5 |
| Evaluation | | | | | | | |
| Type-A durometer hardness | | | 54 | 47 | 51 | 47 | 55 |
| Leaching substance test | Before sterilization | | ○ | ○ | ○ | ○ | ○ |
| | After EOG sterilization | | × | - | × | × | ○ |
| | After steam sterilization | | ○ | ○ | ○ | ○ | ○ |
| Oxygen transmission rate | | | ○ | × | ○ | ○ | ○ |
| Water vapor transmission rate | | | ○ | × | ○ | ○ | ○ |
| Ozone resistance | | | × | ○ | × | × | × |

The results for Examples 1 to 5 and Comparative Examples 1 to 5 shown in Tables 2 and 3 indicate that, where the diene rubber and the non-diene rubber are used in combination as the rubber component and the proportion of the diene rubber is not less than 25 parts by mass and not greater than 55 parts by mass based on 100 parts by mass of the total of the rubbers, the nozzle cap has a moderate gas transmission property and, hence, can be quickly sterilized by the EOG sterilization and the steam sterilization, quickly removed of the residues by the deaeration, and quickly dried in a shorter period of time after the steam sterilization. In addition, the nozzle cap is less liable to be loosened due to increase in internal pressure and detached from the nozzle, and has a reduced leaching substance amount before and after the sterilization.

### REFERENCE SIGNS LIST

- 1, 9:: NOZZLE CAP
- 2, 11:: SYRINGE BARREL
- 3, 12:: NOZZLE
- 4:: NEEDLE
- 5, 10:: SYRINGE
- 6, 13:: TUBULAR PORTION
- 7:: NEEDLE STICKING PORTION
- 8, 14:: OPENING
- D₁, D₃:: INNER DIAMETER
- D₂, D₄:: OUTER DIAMETER
- L₁, L₂:: DIMENSION
- T₁, T₂:: THICKNESS

## Claims

1. A nozzle cap for a prefilled syringe, the nozzle cap being formed from a rubber composition which comprises a rubber component including a diene rubber and a non-diene rubber, wherein the diene rubber is present in a proportion of not less than 20 parts by mass and not greater than 70 parts by mass based on 100 parts by mass of a total of the rubbers in the rubber composition.

2. The nozzle cap according to claim 1, wherein the diene rubber is at least one selected from the group consisting of an isoprene rubber, a butadiene rubber and a styrene butadiene rubber.

3. The nozzle cap according to claim 1 or 2, wherein the non-diene rubber is at least one selected from the group consisting of a butyl rubber and an ethylene propylene rubber.

4. The nozzle cap according to claim 3, wherein the butyl rubber is at least one selected from the group consisting of an isobutylene-isoprene rubber, a chlorinated isobutylene-isoprene rubber, a brominated isobutylene-isoprene rubber, a brominated isobutylene-p-methylstyrene copolymer rubber and a polyisobutylene.

5. The nozzle cap according to any one of claims 1 to 4, wherein the diene rubber is the butadiene rubber and the non-diene rubber is the chlorinated isobutylene-isoprene rubber, wherein the rubber composition further comprises a crosslinking agent including a peroxide crosslinking agent and a triazine crosslinking agent.

6. The nozzle cap according to any one of claims 1 to 5, which has a type-A durometer hardness of not less than 30 and not greater than 70 as measured in conformity with Japanese Industrial Standards JIS K6253-3_{:2012} "Rubber, vulcanized or thermoplastic - Determination of hardness - Part 3: Durometer method."

7. The nozzle cap according to any one of claims 1 to 6, which has an oxygen transmission rate of not less than 5.8 × 10⁻⁹ cm³/ (cm²·s·Pa) and not greater than 2.32 × 10⁻⁸ cm³/ (cm²·s·Pa) as measured per 1-mm thickness of a sample by Method B (isotactic pressure method) specified in Japanese Industrial Standards JIS K7126₋₁₉₈₇ "Gas transmission rate test method for plastic films and sheets."

8. The nozzle cap according to any one of claims 1 to 7, which has a water vapor transmission rate of not less than 0.1 g/(m²·24h) and not greater than 15 g/(m²·24h) as measured per 1-mm thickness of a sample at a temperature of 40°C at a relative humidity of 90 % by a measurement method specified in Japanese Industrial Standards JIS K7129_{:2008} "Plastics - Film and sheeting - Determination of water vapor transmission rate - Instrumental method."

9. The nozzle cap according to any one of claims 1 to 8, which has an ozone resistance such that, even after the nozzle cap is maintained in a test environment having an ozone concentration of 50 ± 5 pphm and a temperature of 40°C as specified in International Standardization Organization Standards ISO1431-1:2004 for 96 hours, the nozzle cap is free from cracking.

10. The nozzle cap according to any one of claims 1 to 9, which includes a minimum thickness portion having a thickness of 1.0 ± 0.5 mm.
